# EUROPEAN PATENT APPLICATION

(11) **EP 4 654 211 A1**
(43) Date of publication of application: **26.11.2025**
(21) Application number: 24177214.4
(22) Date of filing: 22.05.2024
(51) Int. Cl.: G16H 30/20, G16H 40/20, G16H 50/30, A61B 5/16

(54) **MEDICAL IMAGING**

(71) Applicant: Koninklijke Philips N.V., 5656 AG Eindhoven (NL)
(72) Inventor: VAN EE, Raymond, Eindhoven (NL); AZIZ, Gona Nori, Eindhoven (NL); HEUVELINK, Annerieke, 5656AG Eindhoven (NL); HELLE, Michael Günter, Eindhoven (NL); NAUTS, Sanne, Eindhoven (NL)
(74) Representative: Philips Intellectual Property & Standards

(57) **Abstract**

Proposed concepts aim to provide schemes, solutions, concepts, designs, methods, and systems pertaining to a method for predicting relaxation requirements of a subject prior to the subject undergoing a medical imaging procedure. In particular, embodiments aim to provide a method for determining a relaxation index of a subject describing a prediction of a requirement for the subject to be calmed before undergoing the medical imaging procedure based on a determined subject state and a determined subject characteristic.

## Description

### FIELD OF THE INVENTION

This invention relates to the field of medical imaging procedures, and more specifically, to the field of improving the experience of a subject undergoing a medical imaging procedure.

### BACKGROUND OF THE INVENTION

Magnetic Resonance (MR) imaging is a non-invasive diagnostic technique that provides detailed images of the internal structures of the body. It is widely used in the medical field for the diagnosis and monitoring of various diseases and conditions. However, the process of undergoing an MR scan can be stressful for patients, particularly for children and individuals with claustrophobia or other anxiety disorders. This stress can lead to motion artifacts during the scan, which can decrease the quality of the images obtained and potentially affect the accuracy of the diagnosis. Other medical imaging procedures (e.g., CT scans) may suffer from similar effects.

In some instances, it may therefore be required for the patient to be anaesthetized or in some way calmed before they undergo a medical imaging procedure in order that they remain stationary for the entire duration of the scan. The decision to administer anesthesia or sedation to a patient undergoing a medical imaging procedure is typically made by medical staff based on their assessment of the patient's condition and the expected level of stress. This decision is not straightforward and can have a direct impact on the success of the medical exam. If the patient is not properly sedated, they may move during the scan, leading to motion artifacts and potentially requiring the scan to be repeated. On the other hand, unnecessary sedation can lead to increased costs and potential risks for the patient. There is currently no standardized method for predicting the type and level of anesthesia or relaxation a patient may require for an upcoming medical imaging procedure.

### SUMMARY OF THE INVENTION

The invention is defined by the claims.

According to examples in accordance with an aspect of the invention, there is provided a computer-implemented method for predicting relaxation requirements of a subject prior to the subject undergoing a medical imaging procedure.

The method comprises: determining a subject state describing a physiological of psychological state of the subject; determining a subject characteristic describing a physiological or psychological characteristic of the subject; and determining, based on the determined subject state and the determined subject characteristic, a relaxation index describing a prediction of a requirement for the subject to be calmed before undergoing the medical imaging procedure.

Proposed concepts thus aim to provide schemes, solutions, concepts, designs, methods, and systems pertaining to a method for predicting relaxation requirements of a subject prior to the subject undergoing a medical imaging procedure. In particular, embodiments aim to provide a method for determining a relaxation index of a subject describing a prediction of a requirement for the subject to be calmed before undergoing the medical imaging procedure based on a determined subject state and a determined subject characteristic.

Thus, in the proposed invention information is determined about the current state of the patient at a point in time prior to them undergoing a medical imaging procedure. This includes information about a physiological or psychological state of the patient, for example the patient's level of physical or psychological discomfort prior to the medical imaging procedure. The subject state may thus be indicative of how the upcoming medical procedure is affecting the subject's emotional or physical state to be different than their base line normative state. The subject state is then combined with a determined subject characteristic to determine the relaxation index of the subject. The subject characteristic differs from the information described by the subject state in that it describes long-term or intrinsic characteristics of the subject, independent of the effects of the upcoming medical imaging procedure on the emotional state of the subject. The subject characteristic may include information such as the subject's personality or any pre-existing medical conditions they may have.

The proposed invention may thus be advantageous in that it provides a standardized and accurate prediction of relaxation requirements of a subject by leveraging information about both the state of the subject at a point in time prior to the imaging procedure being carried out and contextual information about the subject which may provide further insights into how the subject will behave when subjected to the medical imaging procedure.

The determined relaxation index can be used to improve the likelihood that high quality images can be obtained via the medical imaging procedure. Based on the determined relaxation index of the subject different actions may be taken to calm the patient. This may include administering anesthesia to patients determined to have high stress levels and therefore be very likely to move during the medical imaging procedure and thereby introduce motion artifacts into the medical images. For less extreme cases the subject may simply be exposed to calming influences, such as sounds or lights, prior to undergoing the medical imaging procedure in order to improve their state of distress. In other instances, the relaxation index may be used to determine whether to change parameters of the medical imaging procedure to make it more motion-robust or to reduce the procedure duration for example.

Therefore, the proposed method is advantageous in that it allows for an accurate prediction of relaxation requirements of a subject prior to undergoing a medical imaging procedure leveraging both a subject state and a subject characteristic.

Ultimately, an improved method for predicting relaxation requirements of a subject prior to the subject undergoing a medical imaging procedure may be provided by the proposed concept(s).

In some embodiments, the relaxation index may comprise an anesthesia index describing a prediction of a requirement for anesthesia to be administered to the subject before they undergo the medical imaging procedure. Predicting the type and level of anesthesia to give the subject may be particularly beneficial as the impact of an incorrect decision to anaesthetize may be great.

In some embodiments, the relaxation index may comprise information about a recommended calming treatment to be given to the subject, the calming treatment comprising at least one of: playing music; displaying images; guided breathing; and meditation. Thus, the relaxation index may be used advantageously to provide recommendations of methods or techniques of calming the subject down, allowing for a high quality image of the subject to be taken without the need to sedate the patient.

In some embodiments, the method may further comprise determining a characteristic of the medical imaging procedure, and wherein determining the relaxation index is further based on the determined characteristic of the medical imaging procedure. Different medical imaging procedures may be more or less impacted by motion artifacts caused by fidgeting of the patient within the imaging scanner, therefore using a characteristic of the medical imaging procedure may result in a more accurate determination of whether the subject needs to be calmed before undergoing the imaging procedure.

In some embodiments, the characteristic of the medical imaging procedure may comprise at least one of: a type of medical imaging procedure; a facility at which the medical imaging procedure is to be carried out; a duration of the medical imaging procedure; information about scheduling of the imaging procedure; and a body part of the subject to be imaged. Each of these characteristics may impact how still the patient is required to be for the medical imaging procedure to be successful and/or how stressful the medical imaging procedure is likely to be for the patient. Therefore, including any of these parameters in the determination of the relaxation of the subject may result in a more accurate prediction of a requirement of the subject to be calmed before the medical imaging procedure.

In some embodiments, determining the subject state may be based on at least one of: physiological data of the subject; vital signs data of the subject; a subject self-report of the emotional state of the subject; and a care-giver report of the emotional state of the subject. Each of these forms of data may facilitate the easy and accurate determination of the subject state prior to the scheduled medical imaging procedure.

In some embodiments, the subject state may describe a level of physical or psychological discomfort of the subject. The level of discomfort the subject is in may be a key indicator of whether or not they will move during the medical imaging procedure and therefore its use may improve the accuracy of the relaxation index determination.

In some embodiments, the physiological characteristic of the subject may comprise at least one of: a weight of the subject; a pre-existing medical condition of the subject; and a level of fitness of the subject. Each of these characteristics may contain information about how well the subject is likely to handle the stress of the medical imaging procedure and how well they may respond to different calming treatments or the administration of anesthesia, allowing for reliable determination of the requirement for the subject to be calmed.

In some embodiments, the physiological characteristic of the subject may comprise at least one of: a pre-existing psychological condition of the subject; and a personality of the subject. Personality type and psychological disorders may influence how an individual will react to the potentially stressful environment of a medical imaging procedure. Utilizing this information within an assessment of the relaxation index may result in a greater confidence that the index reflects the individual characteristics of the subject.

In some embodiments, the subject characteristic may comprise information about historic behavior of the subject during a historic medical imaging procedure. Past behavior of the subject may be a key indicator of future behavior and therefore utilizing this information may result in a more reliable method for determining the relaxation index.

In some embodiments, the method may further comprise determining a demographic characteristic of the subject, and determining the relaxation index of the subject may further be based on the determined demographic characteristic of the subject. This allows for demographic factors to be taken into account when predicting the patient's relaxation requirements, potentially improving the accuracy of the prediction.

In some embodiments, the demographic characteristic may comprise at least one of: an age; a gender; and an ethnicity. This provides a comprehensive set of demographic factors that can influence the patient's relaxation requirements, allowing for a more accurate prediction.

In some embodiments, the method may further comprise determining an additional medical procedure value of the subject describing whether the subject is due to undergo any additional medical procedures, and determining the relaxation index of the patient may further be based on the determined additional medical procedure value of the subject. Information about whether the subject is due to undergo any other medical procedures on the same day as the medical imaging procedure may be used to guide the indication of the type and level of anesthesia and/or sedation required by the subject.

According to another aspect of the invention there is provided a computer program comprising computer program code means adapted, when said computer program is run on a computer, to implement the method of any of the previous described embodiments.

According to yet another aspect of the proposed invention, there is provided a system for predicting relaxation requirements of a subject prior to the subject undergoing a medical imaging procedure, the system comprising a processing arrangement configured to: determine a subject state describing a physiological or psychological state of the subject; determine a subject characteristic describing a physiological or psychological characteristic of the subject; and determine, based on the determined subject state and the determined subject characteristic, a relaxation index describing a prediction of a requirement for the subject to be calmed before undergoing the medical imaging procedure.

Thus, there may be proposed concepts for a method for prediction relaxation requirements of a subject prior to the subject undergoing a medical imaging procedure, based on both a determined subject state and a determined subject characteristic.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for predicting relaxation requirements of a subject according to a proposed embodiment;
Fig. 2 is a simplified flow diagram of a method for predicting relaxation requirements of a subject according to an alternative proposed embodiment;
Fig. 3 is a simplified block diagram of a system for predicting relaxation requirements of a subject according to an aspect of the proposed invention: and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purpose of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to predicting relaxation requirements of a subject prior to the subject undergoing a medical imaging procedure. According to proposed concepts, a number of possible solutions may be implemented separately or jointly. That is, although these possible solutions may be described below separately, two or more of these possible solutions may be implemented in one combination or another.

Embodiments of the invention aim to provide a method for predicting relaxation requirements of a subject prior to the subject undergoing a medical imaging procedure. This is achieved by determining both the state of the subject prior to undergoing the medical imaging procedure and a characteristic of the subject and then determining a relaxation index of the subject based on these two values. The relaxation index of the subject describes a prediction that the subject will need to be calmed prior to undergoing the medical imaging procedure.

To obtain high quality images in medical imaging procedures (such as MR scans) it is necessary for the subject to remain still for the duration of the imaging procedure. This ensures that the resulting images do not contain movement artifacts which may obscure certain features depicted in the image and render the image unusable for diagnosis and assessment of the patient. The presence of motion artifacts may result in the imaging procedure having to be repeated, resulting in an inefficiency in the use of the medical imaging scanner, and the subject being exposed to an increased dose.

For certain patients, it may be difficult to ensure that they don't move during the imaging procedure. For example, patients that are particularly stressed about the upcoming procedure, young patients, or patients with certain conditions such as ADHD or Parkinson's disease may find it difficult to remain still. For these patients it may be recommended to perform some kind of calming procedure prior to or during the medical imaging procedure to reduce the likelihood of the patient moving. This could involve giving the patient anesthesia. It may be difficult for medical professionals to decide whether such measures are required. Making the wrong decision about this may impact the through put of the medical imaging scanner and the quality of medical images obtained.

In summary, the proposed solution involves the generation of a relaxation index of the subject describing a prediction of a requirement to calm the subject prior to the subject undergoing a medical imaging procedure. This index is generated based on various signals and data collected from and about the subject and the medical imaging scanner. Crucially, the method involves the determination of two key aspects of the subject:
A. Subject State - this aspect comprises information about the current physiological and psychological state of the subject at some point of time close to the scheduled time of the medical imaging procedure for example on the same day as the imaging procedure; and
B. Subject Characteristic - this aspect comprises information about intrinsic or historic characteristics of the subject that may be indicative of how the subject is likely to react to the medical imaging procedure.

The determined relaxation index may be used by medical professionals to decide whether any calming of the subject is required. The use of this standardized measure may improve the efficiency and effectiveness of medical imaging procedures.

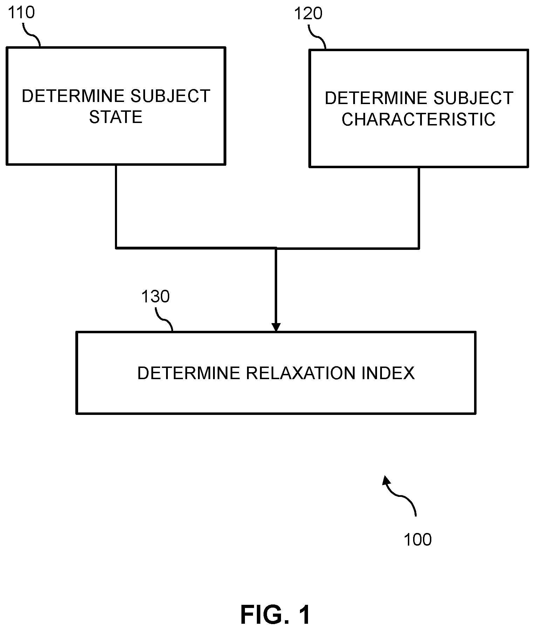

Referring now to Fig. 1, there is depicted a simplified flow diagram of a method 100 for predicting relaxation requirements of a subject according to a proposed embodiment.

The method commences with a step 110, comprising determining a subject state describing a physiological or psychological state of the subject. In this exemplary embodiment, determining the subject state is based on a subject-self report of the emotional state of the subject. The subject due to undergo the medical imaging procedure is given a questionnaire containing a series of questions asking them about their mood and how they feel about the upcoming exam. The results to this questionnaire are used to determine the current emotional state of the subject at a point in time prior to the subject undergoing the medical imaging procedure. In this exemplary embodiment, the subject state comprises a single value describing the level of physical or psychological discomfort of the subject (this may be any suitable quantitative or qualitative measure e.g., a numerical value on a predefined scale of levels of discomfort, or a qualitative categorization of the discomfort level of the patient).

If the subject provides their answers to the questionnaire in written text, Natural Language Processing may be used to determine the level of physical or emotional discomfort the subject is in and determine a value describing the subject state accordingly. Alternatively, through the questionnaire, the individual may be asked to rank various aspects of their physiological and psychological state (e.g., whether they are shaking, how anxious they feel, whether they feel confident they can stay still for the entire imaging procedure, how much pain they are in) on a scale of 1-10. The plurality of rank values thereby obtained, may then be used to determine a numerical measure of the level of physical or psychological discomfort of the subject which is taken as the subject state.

The method further comprises, in a step 120 determining a subject characteristic describing a physiological or psychological characteristic of the subject. In contrast to the subject state, the subject characteristic comprises information about behaviors or traits of the subject over the long-term, not only their current reaction to the upcoming medical imaging procedure. In this exemplary embodiment, the subject characteristic comprises a description of the personality of the subject. The subject's personality is given a rating value in five personality trait categories comprising: agreeableness; conscientiousness; extraversion; neuroticism; and openness to experience, and these five values comprise the subject characteristic. These have been shown to be indicative of how an individual will handle new or potentially stressful circumstances and how they will interact with others. This personality determination of the user may be assessed by a medical professional, or self-assessed by the subject at any time prior to the subject undergoing the medical imaging procedure.

Once the subject state and the subject characteristic have been determined the method proceeds to a step 130 comprising determining, based on the determined subject state and the determined subject characteristic, a relaxation index describing a prediction of a requirement for the subject to be calmed before undergoing the medical imaging procedure. In this exemplary embodiment the relaxation index comprises an anesthesia index describing a prediction of a requirement for anesthesia to be administered to the subject before they undergo the medical imaging procedure. This may contain recommendations as to the type and level of anesthesia to be administered.

There are various ways in which the information described in the subject state and the subject characteristic may be used to determine the relaxation index of the subject. In this exemplary embodiment, determining the relaxation index of the subject comprises comparing the determined subject state to a predetermined state threshold; comparing the determined subject characteristic to a predetermined characteristic threshold; and determining the relaxation index based on these comparisons.

Although in the method 100 determining the subject state is based on a subject-self report of the emotional state of the subject, in other embodiments of the present invention this need not be the case. For example, determining the subject state may be based on at least one of: physiological data of the subject; vital signs data of the subject; and a care-giver report of the emotional state of the subject. Thus, various sources of objective and subjective data may be used to determine the physical and emotional state of the subject prior to the subject undergoing the medical imaging procedure. In this way, the subject state may comprise information about the impact the upcoming imaging procedure is having on the state of the patient. This may be described by the subject state in a qualitative or quantitative manner and comprise one or more values.

Similarly, other aspects of the method 100 may be implemented differently in alternative embodiments. For example, the subject characteristic need not describe the personality of the subject but may describe a different physiological or psychological characteristic of the subject. The physiological characteristic of the subject may comprise at least one of: a weight of the subject; a pre-existing medical condition of the subject; and a level of fitness of the subject. The weight and level of fitness of the subject may impact how the subject may respond to anesthesia or other calming/sedation procedures that may be recommended to be given to the user prior to the medical imaging procedure to increase the likelihood of successful results from the imaging procedure. Pre-existing physical medical conditions such as Parkinson's disease which is associated with the patient experiencing tremors, or medical conditions which result in the subject being subject to pain, may greatly impact the patient's ability to remain still during the imaging procedures. Other medical conditions may be important factors as they influence the subject's suitability to be administered anesthesia. The psychological characteristic of the subject may comprise a pre-existing psychological condition of the subject such as anxiety, ADHD, and autism. Alternatively (or in addition), the subject characteristic may comprise information about historic behavior of the subject during a historic imaging procedure.

In yet other embodiments the subject characteristic may comprise an indication of whether the patient is in control of their own actions. For some patients, such as children under the age of two, or patients with heavy dementia or significant psychological conditions, it may not be possible to guide the individual to stay still during the medical imaging procedure as they are unable to understand and/or respond to any guidance or instructions given to them by a health care professional. The subject characteristic may therefore comprise a value indicating how likely the subject will be able to respond to guidance during the medical imaging procedure.

The proposed method need not result in the determination of anesthesia index, instead the relaxation index may comprise information about a suitability of various different calming treatments to be given to the subject prior to or during the medical imaging procedure. These calming treatments may comprise at least one of: playing music; displaying images; guided breathing; and meditation.

Determining the relaxation index of the subject need not involve the use of predetermined thresholds for the subject state and the subject characteristic as described above. Various other algorithms and logics may be used to predict the requirement for the subject to be calmed before undergoing the medical imaging procedure based on the determined subject state and the determined subject characteristic. Establishing such algorithms may involve the use of historic data describing the subject state and subject characteristics of a plurality of different subjects who underwent medical imaging procedures in the past, and the outcomes of those procedures.

In some embodiments, a machine-learning algorithm may be used to determine the relaxation index from the subject state and the subject characteristic. A machine-learning algorithm is any self-training algorithm that processes input data in order to produce or predict output data. Here, the input data would be the determined subject state and the determined subject characteristic and the output data would be a relaxation index of the subject describing a prediction of a requirement for the subject to be calmed before undergoing the medical imaging procedure.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Purely by way of example, the machine learning model may employ a Convolutional Neural Network, because CNNs have been proven to be particularly successful at analyzing images, with a much lower error rate than other types of neural network. For the specific application of image inpainting problems: U-Nets, GANs, and Diffusion Probabilistic Models have also been shown to be particularly useful.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformations (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In the context of the present invention the training input data entries for a machine learning model correspond to the subject state and the subject characteristic as determined based on historic patient data of different subjects or simulated subject data. The training output data entries correspond to precalculated relaxation indices for these historic or simulated subjects. The precalculated relaxation indices may calculated by a clinician based on their professional medical opinion. In other words, the machine learning model may be trained using a deep learning algorithm configured to receive an array of training inputs and respective known outputs, wherein a training input comprises a subject state and a subject characteristic for a subject and a respective known output comprises a known relaxation index for the subject. In this way, the machine learning model may be trained to automatically determine a relaxation index for a subject due to undergo a medical imaging procedure based on a subject state describing the physiological or psychological state of the subject and a subject characteristic describing a physiological or psychological characteristic of the subject.

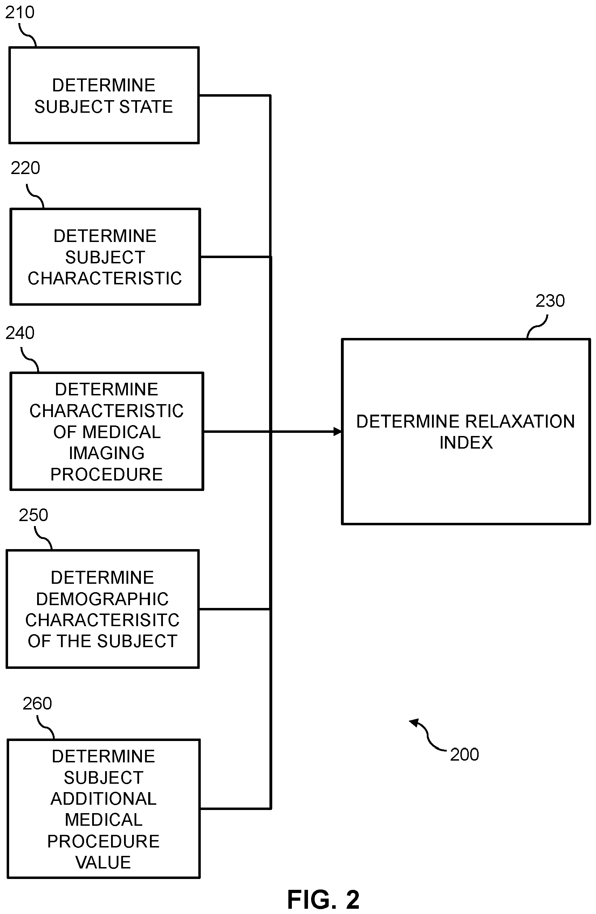

Referring now to Fig. 2 there is depicted a simplified flow diagram of a method 200 for predicting relaxation requirements of a subject according to an alternative proposed embodiment.

The steps 210 and 220 of the method 200 are substantially the same as the steps 110 and 120 of the method 100 described above, and therefore a description of these steps is not repeated. The method 200 differs from the method 100 in that it further comprises a step 240 of determining a characteristic of the medical imaging procedure, a step 250 of determining a demographic characteristic of the subject, and a step 260 of determining a subject additional medical imaging procedure value.

The characteristic of the medical imaging procedure in this example comprises at least one of: a type of medical imaging procedure; a facility at which the medical imaging procedure is to be carried out; a duration of the medical imaging procedure; information about scheduling of the medical imaging procedure; and a body part of the subject to be imaged. In this exemplary embodiment, the demographic characteristic of the individual comprises at least one of: an age; a gender; and an ethnicity. The additional medical procedure value describes whether the subject is due to undergo any additional medical procedures and information about any such procedures, for example the time they are scheduled for and whether they require anesthesia.

Once the subject state, the subject characteristic, the characteristic of the medical imaging procedure, and the demographic characteristic of the subject have been determined the method proceeds to a step 230 comprising determining, based on the subject characteristic, the characteristic of the medical imaging procedure, the demographic characteristic of the subject, and the subject additional medical procedure value, a relaxation index of the subject describing a prediction of a requirement for the subject to be calmed before undergoing the medical imaging procedure.

The method 200 may be advantageous in that it leverages several sources of contextual information to generate the relaxation index of the subject. The details of the exact medical imaging procedure being carried out may greatly affect an individual's reaction to undergoing the procedure. Some procedures may be inherently more stressful than others (e.g. may involve more constrictive imaging bores or involve louder more intrusive noises) and some imaging procedures may be more sensitive to motion artifacts than others. Whether or not the subject is due to undergo another medical procedure may impact the level of stress the subject may be experiencing. Also, if the subject is due to undergo a further medical procedure on the same day as the medical imaging procedure, this may also be taken into account into any prediction made as to whether to give the subject anesthesia. If the subject is undergoing a medical procedure requiring anesthesia later in the day this may influence any recommendation of the type and level of anesthesia to be administered to the patient prior to them undergoing the medical imaging procedure.

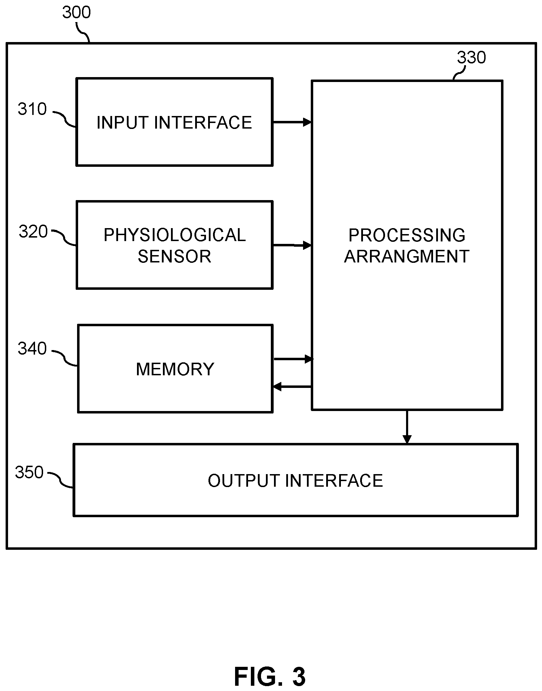

Referring now to Fig. 3 there is depicted a system 300 for predicting relaxation requirements of a subject prior to the subject undergoing a medical imaging procedure according to an aspect of the present invention. The system 300 comprises an input interface 310, at least one physiological sensor 320, a processing arrangement 330, a memory 340, and an output interface 350.

The input interface 310 may be used by a subject before they undergo a medical imaging procedure to input data related to how they feel and their current emotional and physical state. This may involve the user filling out a questionnaire containing questions about the subject's level of physical and emotional discomfort. Alternatively, the input interface may be used by a clinician to input their assessment of how the subject is behaving and how they appear to be coping with the prospect of the upcoming medical imaging procedure. Any data input into the system 300 via the input interface 310 is transmitted to the processing arrangement 330.

The at least one physiological sensor 320 may be used to obtain physiological data of the subject. For example, the system 300 may comprise vital signs sensors to measure the heart rate, blood pressure, and breathing rate of the subject at a point in time close to when the subject is due to undergo the medical imaging procedure. This may point to the physical state of the patient or may indicate their emotional state. For example, fast breathing and heart rate combined with high blood pressure may indicate that the subject is undergoing significant stress due to the upcoming medical imaging procedure. Other examples of sensors that may be present in the system 300 include wearable technology, noncontact sensors or video monitoring devices. Video footage of the subject may be used to monitor the motion of the subject's hands, trembling of their body, or body gait aspects - all of which may be indicative of the physiological and psychological state of the subject. One physiological data has been obtained by the at least one physiological sensor 320, it is transmitted to the processing arrangement 330.

The processing arrangement then determines, based on the data gathered by the input interface and the at least one physiological sensor, a subject state describing a physical or physiological state of the subject.

The processing arrangement 330 is associated with a memory 340. Stored in the memory 340 is information comprising the medical history of the patient, past questionnaire data of the patient, demographic information of the patient, and the personality of the patient i.e. the memory 340 may form part of a hospital's electronic health record system. The processing arrangement 330 is configured to access the subject data stored within the memory 340 to determine a subject characteristic describing a physiological or psychological characteristic of the subject.

Once the subject state and the subject characteristic have been determined, the processing arrangement 330 is further configured to determine, based on the subject characteristic and the subject state a relaxation index of the subject describing a prediction of a requirement for the subject to be calmed before undergoing the medical imaging procedure. This may then be output to a clinician or health care professional via the output interface 350. The determined relaxation index may also be stored in the memory 340 to allow the value to be accessed by a health care professional at a future point in time.

The system 300 may be further equipped with a feedback mechanism such that when new data is input into the hospital's electronic health record system, or further inputs are provided via the input interface 310, the system 300 may be configured to automatically update the value of the relaxation index of the subject based on the new data.

The determined relaxation index may be used by the clinician in various ways. It may be used to decide whether to administer to the subject anesthesia and/or sedation, and if so the level and type of anesthesia or sedation. Alternatively, the relaxation index may indicate to the clinician to attempt to calm the subject using relaxing measures. In other examples, the relaxation index may be used to adjust the workflow protocol for the medical imaging procedure. For example, it may be determined to use very fast scan sequences to keep the procedure time as short as possible or to use motion-robust scans to reduce the impact of the subject's motion during the medical imaging procedure. In some instances, it may be decided to adjust the walking route for the patient through the hospital, to avoid scenes that may be particularly stress inducing.

Thus, the relaxation index may be a single value indicating the likelihood calming measures are required prior to the subject undergoing the medical imaging procedure based on which a health care professional may decide to subject the individual to different calming treatments, administer an aesthetic, or adjust parameters of the medical imaging procedure. Alternatively, the relaxation index itself comprise recommendations of further actions to be taken including any of those discussed above. All this information may be displayed to a health care professional via the output interface 350 of the system 300.

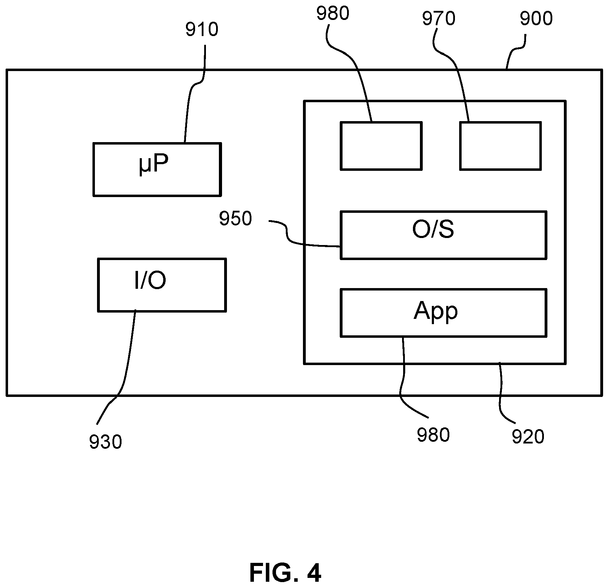

Fig. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A computer-implemented method (100) for predicting relaxation requirements of a subject prior to the subject undergoing a medical imaging procedure, the method comprising:
determining (110) a subject state describing a physiological or psychological state of the subject;
determining (120) a subject characteristic describing a physiological or psychological characteristic of the subject; and
determining (130), based on the determined subject state and the determined subject characteristic, a relaxation index describing a prediction of a requirement for the subject to be calmed before undergoing the medical imaging procedure.

2. The method of claim 1, wherein the relaxation index comprises an anesthesia index describing a prediction of a requirement for anesthesia to be administered to the subject before they undergo the medical imaging procedure.

3. The method of claim 1, wherein the relaxation index comprises information about a recommended calming treatment to be given to the subject, the calming treatment comprising at least one of:
playing music;
displaying images;
guided breathing; and
meditation.

4. The computer-implemented method of claim 1, wherein the method further comprises determining (240) a characteristic of the medical imaging procedure, and
wherein determining (230) the relaxation index is further based on the determined characteristic of the medical imaging procedure.

5. The computer-implemented method of claim 4, wherein the characteristic of the medical imaging procedure comprises at least one of:
a type of medical imaging procedure;
a facility at which the medical imaging procedure is to be carried out;
a duration of the medical imaging procedure;
information about scheduling of the medical imaging procedure; and
a body part of the subject to be imaged.

6. The computer-implemented method of any preceding claim, wherein determining the subject state is based on at least one of:
physiological data of the subject;
vital signs data of the subject;
a subject self-report of the emotional state of the subject; and
a care-giver report of the emotional state of the subject.

7. The computer-implemented method of any preceding claim, wherein the subject state describes a level of physical or psychological discomfort of the patient.

8. The computer-implemented method of any preceding claim, wherein a physiological characteristic of the subject comprises at least one of:
a weight of the subject;
a pre-existing medical condition of the subject; and
a level of fitness of the subject.

9. The computer-implemented method of any preceding claim, wherein a psychological characteristic of the subject comprises at least one of:
a pre-existing psychological condition of the subject; and
a personality of the subject.

10. The computer-implemented method of any preceding claim, wherein the subject characteristic comprises information about historic behavior of the subject during a historic medical imaging procedure.

11. The computer-implemented method of any preceding claim, wherein the method further comprises determining (250) a demographic characteristic of the subject, and
wherein determining (230) the relaxation index of the subject is further based on the determined demographic characteristic of the subject.

12. The computer-implemented method of claim 11, wherein the demographic characteristic comprises at least one of:
an age;
a gender;
an ethnicity.

13. The computer-implemented method of any preceding claim, wherein the method further comprises determining (260) an additional medical procedure value of the subject describing whether the subject is due to undergo any additional medical procedures, and
wherein determining (230) the relaxation index of the patient may further be based on the determined additional medical procedure value of the subject.

14. A computer program comprising computer program code means adapted, when said computer program is run on a computer, to implement the method of any of claims 1-12.

15. A system (300) for predicting relaxation requirements of a subject prior to the subject undergoing a medical imaging procedure, the system comprising:
A processing arrangement (330) configured to:
determine a subject state describing a physiological or psychological state of the subject;
determine a subject characteristic describing a physiological or psychological characteristic of the subject; and
determine, based on the determined subject state and the determined subject characteristic, a relaxation index describing a prediction of a requirement for the subject to be calmed before undergoing the medical imaging procedure.
